(19) Europäisches Patentamt

European Patent Office

Office européen des brevets

(11) EP 0 609 127 B1

(12) **FASCICULE DE BREVET EUROPEEN**

(45) Date de publication et mention
de la délivrance du brevet:
**27.12.1996 Bulletin 1996/52**

(51) Int Cl.6: **C07C 67/08**, C07C 69/54

(21) Numéro de dépôt: **94400143.7**

(22) Date de dépôt: **24.01.1994**

(54) **Procédé perfectionné de fabrication de (méth)acrylates d'alkyle par estérification directe**

Verfahren zur Herstellung von Alkyl-(meth)acrylaten durch direkte Veresterung

Process for the production of alkyle (meth)acrylates by direct esterification

(84) Etats contractants désignés:
**AT BE CH DE DK ES FR GB GR IE IT LI LU NL PT SE**

(30) Priorité: **27.01.1993 FR 9300827**

(43) Date de publication de la demande:
**03.08.1994 Bulletin 1994/31**

(73) Titulaire: **ELF ATOCHEM S.A.**
**92800 Puteaux (FR)**

(72) Inventeurs:
• **Fauconet, Michel**
  **F-57730 Valmont (FR)**
• **Richard, Norbert**
  **F-57150 Creutz Wald (FR)**

• **Esch, Marc**
  **F-57800 Freyming-Merlebach (FR)**
• **Colin, Nadine**
  **F-57500 Saint-Avold (FR)**

(74) Mandataire: **Chaillot, Geneviève**
**Cabinet CHAILLOT,**
**B.P. No. 74**
**92703 Colombes Cédex (FR)**

(56) Documents cités:
**EP-A- 0 463 434**

• **PATENT ABSTRACTS OF JAPAN vol. 015, no. 163 (C-0826)1991 & JP-A-30 034 956 (IDEMITSU PETROCHEM CO LTD)**
• **PATENT ABSTRACTS OF JAPAN vol. 015, no. 163 (C-0826)1991 & JP-A-30 034 956**

## Description

La présente invention concerne un procédé perfectionné de fabrication de (méth)acrylates d'alkyle en $C_1$-$C_{15}$, linéaires, ramifiés ou cycliques, par une estérification directe de l'acide (méth)acrylique par l'alcool correspondant, cette réaction étant catalysée par de l'acide sulfurique.

Les problèmes qui se posent dans le cas de cette fabrication vont maintenant être exposés, par commodité, sur la base de l'exemple de l'estérification directe de l'acide acrylique par le butanol. Ces problèmes et la solution proposée par l'invention sont les mêmes dans le cas de l'emploi, d'une part, de l'acide méthacrylique, et, d'autre part, d'alcools autres que le butanol.

Le procédé industriel de fabrication de l'acrylate de butyle met donc en jeu une réaction d'estérification directe de l'acide acrylique par le butanol, réaction catalysée par de l'acide sulfurique. Dans ce procédé, pour déplacer l'équilibre réactionnel, il n'est pas rajouté de solvant entraînant azéotropiquement l'eau de réaction, mais cette fonction est assurée par un excès de l'alcool estérifiant (en l'occurrence, le butanol), qui présente la particularité de constituer un azéotrope avec l'eau.

A l'issue de l'étape réactionnelle réalisée en discontinu, la quasi-totalité de l'acide sulfurique a été transformée en sulfate acide de butyle $BuSO_4H$, selon la réaction suivante d'estérification de l'acide sulfurique par le butanol :

$$BuOH + H_2SO_4 \rightarrow HBuSO_4 + H_2O$$

Par conséquent, le mélange réactionnel, en fin de réaction, comprend de l'acrylate de butyle, du butanol, de l'acide acrylique, du sulfate acide de butyle, des traces d'acide sulfurique, et les stabilisants classiquement utilisés dans la réaction.

On fait suivre la réaction d'estérification par des étapes de purification, généralement effectuées en continu :

- on neutralise les espèces acides contenues dans le mélange brut réactionnel, par addition à celui-ci d'une solution aqueuse de base (soude) ; pendant cette étape, l'acide acrylique est neutralisé en acrylate de sodium, le sulfate acide de butyle, en sulfate neutre de butyle $BuSO_4Na$, et les traces d'acide sulfurique, en sulfate de sodium $Na_2SO_4$, tous ces sels passant dans la phase aqueuse ;
- on lave à l'eau, dans une colonne d'extraction, la phase organique séparée, issue de la neutralisation, afin d'éliminer les traces d'impuretés, de soude et de sels, puis l'on envoie l'acrylate de butyle lavé sur une première colonne à distiller, permettant d'éliminer en tête les produits légers, lesquels sont recyclés à la réaction d'estérification. On envoie ensuite l'ester étêté sur une deuxième colonne, d'où il sort en tête, purifié des produits lourds ;
- on récupère le butanol présent :

  - dans la phase aqueuse séparée, issue de la neutralisation, laquelle contient essentiellement du sulfate neutre de butyle, du butanol, de l'acrylate de sodium et des traces de sulfate de sodium ;
  - dans la phase aqueuse issue de la réaction d'estérification proprement dite ; ainsi que
  - dans la phase aqueuse issue du lavage dans la colonne d'extraction de la phase organique,

  cette récupération du butanol étant réalisée dans une colonne de distillation, alimentée en tête par les eaux à traiter, dans laquelle le butanol est entraîné en tête et peut être recyclé à la réaction d'estérification, tandis que le pied de colonne constitue les eaux usées, débarrassées de butanol, qui seront rejetées à la station de traitement biologique.

Les eaux ainsi rejetées contiennent une forte pollution organique, mesurée par la demande chimique en oxygène (DCO) à cause de la présence :

- de sulfate neutre de butyle : la DCO théoriquement rejetée est de l'ordre de 2 kg d'$O_2$ par kg d'acide sulfurique mis en jeu en réaction d'estérification ;
- d'acrylate de sodium : la DCO théorique, due à la présence d'acide acrylique résiduel en fin de réaction, est d'environ 8 kg d'$O_2$ par tonne d'acrylate de butyle fabriqué, soit environ le quart de la DCO due au sulfate neutre de butyle ; elle peut être augmentée par une hydrolyse partielle de l'acrylate de butyle au cours des étapes de purification dudit ester.

On pourrait éviter la pollution due au sulfate acide (ou neutre) de butyle en utilisant d'autres catalyseurs acides que l'acide sulfurique, qui possèdent la particularité de ne pas donner de réaction d'alcoolyse avec l'alcool. Par exemple, il est connu que l'acide méthane sulfonique (AMS) et l'acide paratoluène sulfonique (APTS) ne réagissent pas, ou ne réagissent que dans une moindre mesure, avec les alcools, pour former des sulfates d'alkyle. Toutefois, ces catalyseurs ont l'inconvénient d'être beaucoup plus coûteux que l'acide sulfurique, ce qui oblige à opérer une récupération de ces catalyseurs en fin de réaction, en vue de les recycler. Cette récupération, qui pourrait être envisagée par extraction à l'eau, ne convient pas ici, du fait de la présence d'alcool en excès pour sortir l'eau de réaction, cet alcool jouant un rôle de solvant compétitif par rapport à l'eau, et limitant ainsi le rendement d'extraction du catalyseur dans l'eau.

D'autres techniques sont envisageables pour réduire la pollution due au sulfate d'alkyle, mais elles présentent toutes des inconvénients :

- une hydrolyse du sulfate acide d'alkyle peut être réalisée dans le milieu organique réactionnel lui-même en fin de réaction d'estérification, comme il est décrit dans l'article de J. WASILEWSKI, G. KESICKA, H. SMOLIK, E. CHROMIAK, Przemysl Chemiczny, 11-12 60, 1981, 541-543. Dans cet article, les auteurs décrivent l'hydrolyse du sulfate de 2-éthyl hexyle dans le milieu réactionnel final de synthèse de l'acrylate de 2-éthyl hexyle, à 80-105°C, avec 7,5% en poids d'eau, pendant 15 à 30 minutes. Les inconvénients principaux sont :

  - un faible rendement d'élimination du sulfate acide de 2-éthyl hexyle ;
  - l'hydrolyse de l'ester acrylate de 2-éthyl hexyle fabriqué, ce qui provoque une perte de produit et une augmentation de la pollution rejetée sous la forme d'acide acrylique (ou d'acrylate de sodium après neutralisation) ;
  - la nécessité de traiter un flux important, puisque c'est la totalité du mélange réactionnel brut de fin de réaction d'estérification qui est traitée ;
  - un risque de nuire à la qualité et à la stabilité de l'acrylate de 2-éthyl hexyle, qui est un ester polymérisable.

- on peut également envisager l'extraction du sulfate acide de butyle à l'eau, en faisant suivre par une nouvelle extraction avec l'alcool estérifiant, comme cela figure dans la demande de brevet allemand DE-A-4 019 781. Cette solution au problème posé n'est pas applicable dans le cas du procédé de fabrication de (méth) acrylates d'alkyle par estérification directe, car l'excès d'alcool (butanol) joue le rôle de tiers solvant et limite fortement le rendement d'extraction du sulfate acide de butyle dans l'eau. Pour être applicable, il serait nécessaire de mettre en jeu un solvant, ce qui compliquerait le procédé, notamment pour la récupération et le recyclage de ce solvant.

La Société Déposante a donc recherché une nouvelle solution au problème posé, et elle a découvert que l'hydrolyse du sulfate acide de butyle (en général d'alkyle) dans la phase aqueuse de l'étape de neutralisation (où il est présent sous sa forme saline) permet d'améliorer considérablement la pollution rejetée dans les eaux épuisées d'un atelier de fabrication de l'acrylate de butyle : la DCO est réduite d'au moins un facteur 5 (par exemple, comparaison entre l'Exemple 1 de Référence ci-après et l'Exemple 2B de l'invention). Le traitement en station d'épuration présente un coût qui se trouve ainsi réduit. De plus, cette hydrolyse permet de récupérer et de recycler le butanol ayant réagi avec l'acide sulfurique. Ce butanol récupéré, qui était sinon perdu, représente environ 11 kg par tonne d'acrylate de butyle fabriqué. On peut souligner ici qu'une saponification de HBuSO$_4$ sous sa forme saline (NaBuSO$_4$) selon la réaction :

$$NaBuSO_4 + NaOH \rightleftarrows Na_2SO_4 + BuOH$$

conduit à de très mauvais rendements. Ainsi, les rendements d'une saponification (d'après NaOH consommé) d'une solution de NaBuSO$_4$ à 5% en poids dans l'eau par 2 moles de NaOH/mole de NaBuSO$_4$ à 130 °C, étaient de 4,9%, 7% et 8,5% au bout de respectivement 1, 2 et 3 heures.

Le principe du traitement selon l'invention, applicable à d'autres alcools et à l'acide méthacrylique comme cela a été indiqué ci-dessus, consiste donc à hydrolyser le sulfate acide de butyle, de manière à régénérer le butanol et l'acide sulfurique :

$$HBuSO_4 + H_2O \rightarrow BuOH + H_2SO_4$$

Le butanol régénéré est facilement éliminé dans la phase aqueuse issue de ce traitement, par distillation dans la colonne existante. De cette manière toute la pollution imputable au sulfate acide de butyle (plus exactement au sulfate neutre de butyle après neutralisation) est éliminée.

La technique proposée selon l'invention a l'avantage, par rapport à celle qui consiste à hydrolyser le sulfate acide de butyle dans le mélange brut obtenu après estérification, outre une efficacité meilleure, de traiter une phase aqueuse très peu concentrée en ester final polymérisable et hydrolysable, donc d'éviter tout risque de dégrader le rendement de la fabrication et de nuire à la qualité du monomère pur. De plus, le volume engagé dans la réaction d'hydrolyse (flux aqueux mineur par rapport au flux organique principal contenant l'ester) est beaucoup plus faible, et nécessite par conséquent une taille de réacteur réduite.

La présente invention a donc pour objet un procédé de fabrication d'un (méth)acrylate d'alkyle en $C_1$-$C_{15}$ par estérification directe de l'acide (méth)acrylique par l'alcool correspondant, ladite estérification étant catalysée par l'acide sulfurique, le mélange réactionnel brut obtenu comprenant du (méth)acrylate d'alkyle en $C_1$-$C_{15}$, de l'alcool, de l'acide (méth)acrylique, du sulfate acide d'alkyle en $C_1$-$C_{15}$, des traces d'acide sulfurique et les impuretés habituelles, procédé suivant lequel on fait suivre l'estérification par :

(a) l'addition audit mélange réactionnel brut d'une base pour neutraliser l'acide (méth)acrylique, le sulfate acide d'alkyle en $C_1$-$C_{15}$, les traces d'acide sulfurique qui y sont présents, les sels résultants passant dans la phase aqueuse dudit mélange, la phase organique et la phase aqueuse issues de cette neutralisation étant séparées et le (méth)acrylate d'alkyle recherché étant récupéré à partir de ladite phase organique ;
(b) la récupération de l'alcool à partir de ladite phase aqueuse, les eaux usées débarrassées dudit alcool étant rejetées,

caractérisé par le fait qu'on conduit une hydrolyse du sulfate acide d'alkyle présent dans la phase aqueuse issue de la séparation conduite après neutralisation, de manière à former dans ladite phase de l'alcool et de l'acide sulfurique, on neutralise les espèces acides résultant de ladite hydrolyse par introduction d'une base dans le milieu, et on adresse la phase aqueuse résultante à l'étape de récupération de l'alcool.

On peut conduire cette hydrolyse et l'étape de neutralisation qui la suit, en mode continu ou en mode discontinu.

Dans un mode de réalisation particulièrement préféré, on conduit l'hydrolyse en présence d'acide sulfurique comme catalyseur et agent de neutralisation des espèces basiques présentes, ledit acide sulfurique étant introduit dans un rapport molaire $R_H$ du nombre d'équivalents $H^+$ en excès au nombre de moles de sulfate acide d'alkyle au moins égal à 1,5. Pour des raisons de mise en oeuvre industrielle, le rapport molaire $R_H$ est, de préférence, compris entre 1,5 et 2.

Par ailleurs, on conduit avantageusement l'hydrolyse à une température comprise entre environ 70 et 200°C, pendant une durée qui dépend de la température appliquée, la pression étant fonction de la température réactionnelle.

A la sortie du réacteur d'hydrolyse, le milieu réactionnel est neutralisé généralement par l'addition de soude aqueuse dans la conduite de sortie, puis il est refroidi, généralement à une température de l'ordre de 60°C par exemple à travers un échangeur. Cette neutralisation est destinée à éliminer l'acide sulfurique en excès pour éviter la corrosion des appareils en aval.

On conduit l'étape de récupération de l'alcool, de façon connue en soi, dans une colonne de distillation, l'alcool étant entraîné en tête dans ladite colonne.

On conduit l'étape de récupération de l'ester (méth)acrylate également de façon classique en lavant à l'eau, dans une colonne d'extraction, la phase organique issue de la séparation de phases qui suit la première neutralisation, puis en adressant le (méth)acrylate d'alkyle lavé à une première colonne à distiller, pour éliminer en tête, les produits légers, recyclés à la réaction d'estérification, et, enfin, en adressant le (méth)acrylate d'alkyle étêté sur une deuxième colonne à distiller, d'où il sort en tête purifié des produits lourds.

Enfin, on adresse à la colonne de récupération d'alcool précitée :

- la phase aqueuse issue de la réaction d'estérification proprement dite ;
- la phase aqueuse résultant de l'hydrolyse après neutralisation ; et
- la phase aqueuse issue de la colonne d'extraction précitée.

Comme alcool, on peut citer le méthanol, le butanol, l'hexanol, le 2-éthyl hexanol, etc.

Pour mieux illustrer l'objet de la présente invention, on va en décrire ci-après plusieurs exemples de mise en oeuvre, avec référence au dessin annexé, sur lequel

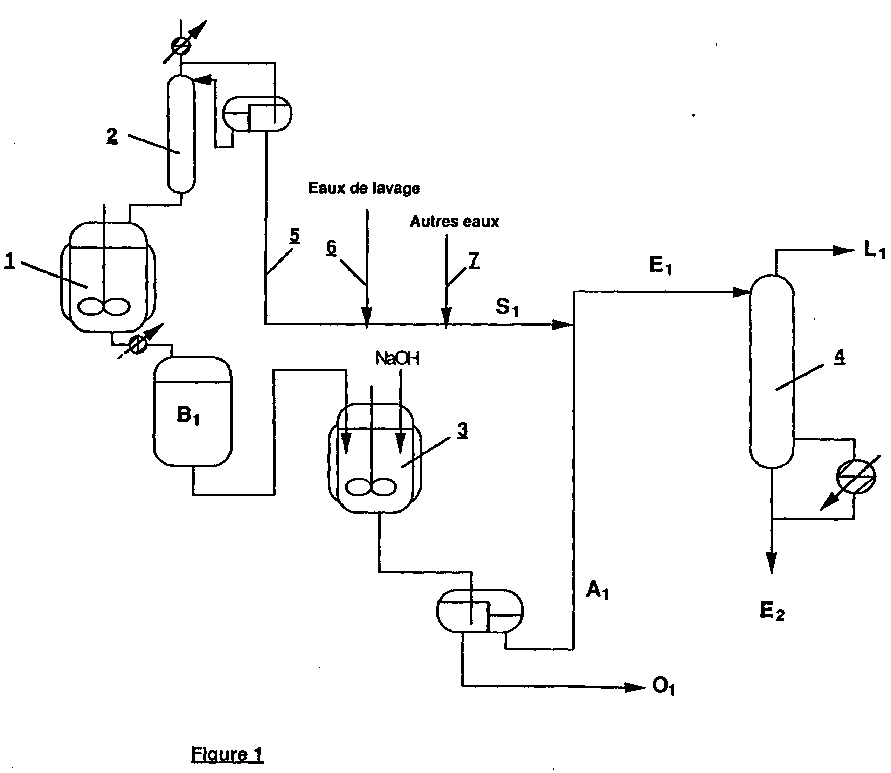

- les Figures 1 à 3 représentent des schémas respectivement du procédé connu pour la fabrication d'acrylate de butyle ou de 2-éthyl hexyle (cf. Exemples respectivement 1 et 5 de référence), d'un procédé de fabrication d'acrylate de butyle ou de 2-éthyl hexyle selon l'invention (cf. Exemples respectivement 2 et 6 de l'invention) et d'un procédé de fabrication d'acrylate de butyle avec hydrolyse du sulfate acide de butyle dans le mélange brut réactionnel (Exemple Comparatif 3) ; et

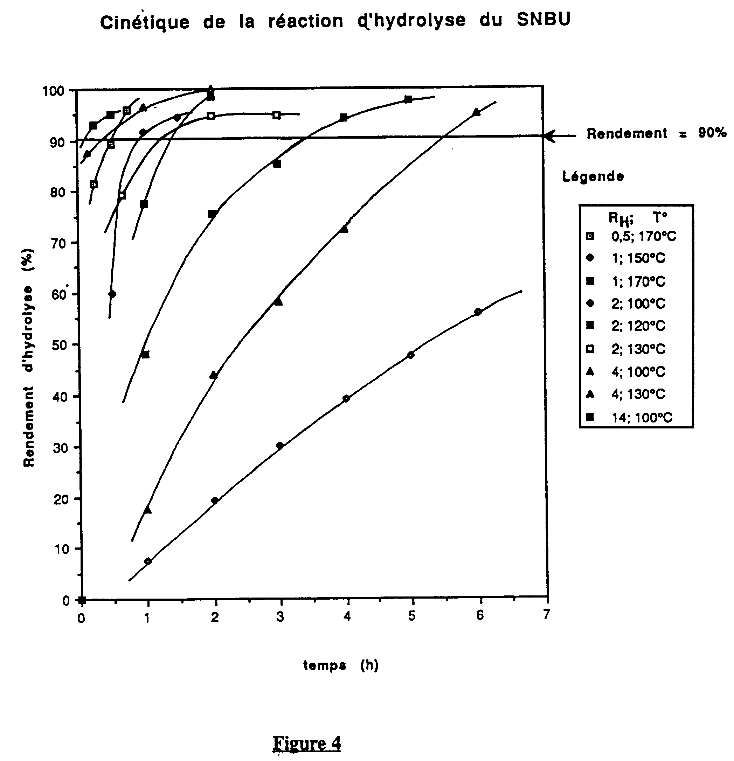

- la Figure 4 illustre la cinétique de la réaction d'hydrolyse du sulfate neutre de butyle, en donnant différentes courbes du rendement d'hydrolyse en fonction du temps, pour diverses valeurs de la température d'hydrolyse et du rapport $R_H$ défini ci-dessus.

Dans ce qui suit, toutes les parties et tous les pourcentages sont exprimés en poids, sauf indication contraire, SABU et SNBU désignant les sulfates respectivement acide et neutre de butyle, et SAEH et SNEH désignant les sulfates respectivement acide et neutre de 2-éthyl hexyle.

Exemple 1 de Référence (Fig. 1) - Fabrication d'acrylate de butyle

A - Synthèse du mélange brut réactionnel :

On conduit, en discontinu, dans le réacteur agité 1, à une température de 80°C, sous pression réduite, une estérification d'acide acrylique (1 partie) par le butanol (1,337 partie), en présence d'acide sulfurique à 94% (0,025 partie) comme catalyseur, et en présence d'inhibiteurs de polymérisation (0,002 partie). L'équilibre de la réaction d'estérification est déplacé vers la formation de l'ester attendu acrylate de butyle par distillation, en 2, de l'eau générée, sous forme d'un mélange azéotropique avec le butanol.

La composition du brut post-réactionnel ($B_1$) est la suivante :

| | |
|---|---|
| acrylate de butyle | : 79-81% |
| butanol | : 12-14% |
| acide acrylique | : 0,1-0,5% |
| sulfate acide de butyle | : 1,7-1,8% |
| acide sulfurique | : 0-0,02% |
| eau | : 0,2-0,3%. |

B - Purification de l'acrylate de butyle

(a) Neutralisation des espèces acides

Le mélange réactionnel ($B_1$) ainsi constitué est neutralisé pour éliminer les acides présents, à savoir le sulfate acide de butyle, l'acide acrylique et les traces d'acide sulfurique. Cette neutralisation a lieu dans le réacteur agité 3, en discontinu ou en continu, avec une solution de soude à 8%, en léger excès (5%), pendant un temps de 1 à 3 minutes, à une température de 20 - 50°C. A l'issue de cette neutralisation, le mélange décante en deux phases : une phase organique ($O_1$) et une phase aqueuse ($A_1$).

(b) Récupération de l'alcool

Lors de la fabrication de l'acrylate de butyle, toutes les eaux usées de l'atelier ($E_1$) sont rassemblées et envoyées en tête d'une colonne de distillation 4, qui permet de séparer les composés légers ($L_1$) (butanol, acrylate de butyle, qui sont récupérés en tête en vue d'être recyclés) des eaux épuisées ($E_2$), lesquelles sont envoyées à la station finale de traitement biologique.

Pour simuler cette partie de la fabrication de l'acrylate de butyle, on a opéré sur une colonne de distillation, assurant une séparation en

- fraction de tête     : 25-27%
- fraction de queue     : 73-75%.

Cette colonne est alimentée en tête, de façon continue, par un mélange représentatif du mélange industriel ($E_1$), constitué par :

- la phase aqueuse ($A_1$), obtenue par la neutralisation du mélange brut ($B_1$) ;
- un mélange synthétique ($S_1$) représentatif des autres eaux usées de l'atelier :

  - eaux générées lors de la réaction d'estérification (symbolisées en 5)
  - eaux de lavage de la phase organique ($O_1$) (symbolisées en 6)
  - autres eaux de l'atelier (symbolisées en 7).

    La composition du mélange ($S_1$) est la suivante :
- eau     : 96,3% ;
- butanol     : 3% ;
- acrylate de butyle     : 0,3% ;
- autres composants     : 0,4%.

Le nouveau mélange ainsi constitué, représentatif du mélange industriel ($E_1$), est composé de 1 partie de ($A_1$)

pour 2,45 parties de $(S_1)$.

C - Résultats

Cet essai de référence est conduit selon le mode opératoire décrit ci-dessus, sans traitement particulier de la pollution rejetée dans les eaux usées.

A l'issue de l'étape réactionnelle, la totalité de l'acide sulfurique engagé a été transformée en sulfate acide de butyle dans le mélange $(B_1)$ et ce composé passe intégralement dans la phase aqueuse $(A_1)$. Après mélange de cette phase aqueuse avec le mélange synthétique $(S_1)$, afin de former le mélange $(E_1)$, les eaux épuisées $(E_2)$ présentent une DCO de 41,7 g d'$O_2$ par kg d'eaux épuisées, dont 70 à 80% sont dus à la présence du sulfate acide de butyle.

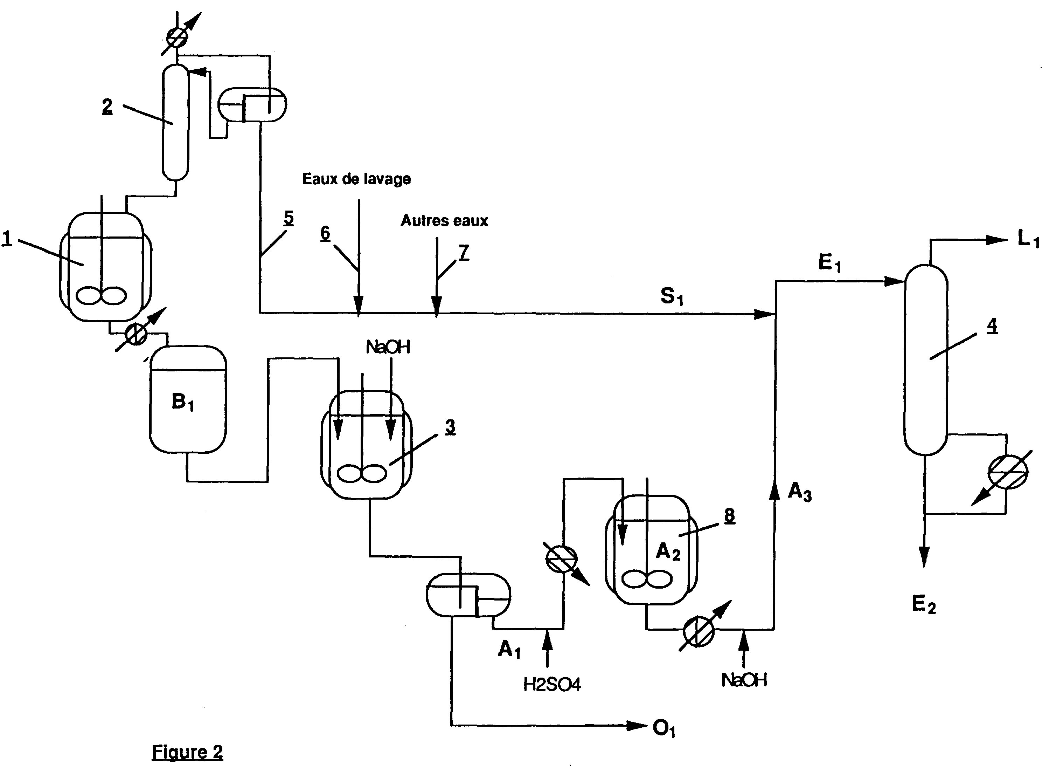

Exemple 2 de l'invention (Fig. 2) - Fabrication d'acrylate de butyle

On procède comme à l'Exemple de Référence 1, mais l'on prévoit l'élimination du sulfate acide de butyle par hydrolyse acide du sulfate neutre de butyle contenu dans la phase aqueuse $(A_1)$. Expérimentalement, cette réaction a été conduite selon deux modes :

A - En discontinu :

La phase aqueuse $(A_1)$ contient principalement du sulfate neutre de butyle, de l'acrylate de sodium, et de la soude en excès. Après dosage de ces trois constituants, on introduit une quantité d'acide sulfurique à 94%, telle que, après neutralisation de la soude en excès et déplacement de l'acide acrylique de son sel acrylate de sodium (ANa), on obtienne le rapport molaire voulu :

$R_H$ = nombre d'équivalents $H^+$ en excès/nombre de moles de sulfate neutre de butyle, soit :
$R_H$ = $(H^+)_{excès}$/(SNBU)

avec : $(H^+)_{excès}$ = $(H^+)_{introduit}$ - (ANa) - $(NaOH)_{libre}$.

Dans un réacteur 8, équipé d'un agitateur mécanique, le mélange $(A_2)$ ainsi constitué est porté sous agitation à une température T° pendant un temps $t_R$.

A l'issue de ce temps, le mélange est refroidi à 70-80 °C, puis neutralisé par NaOH à 8% jusqu'à un pH de 8, pour éliminer les espèces acides . On obtient ainsi un mélange $(A_3)$, qui est ensuite introduit dans l'étape de récupération de l'alcool en 4 en lieu et place du mélange $(A_1)$ de l'Exemple de Référence 1.

Résultats

Pour vérifier en conditions réelles les résultats obtenus sur des mélanges synthétiques de sulfate acide de butyle (Exemple 4), une fabrication d'acrylate de butyle a été simulée, selon les conditions décrites à l'Exemple de Référence 1 (synthèse du mélange brut réactionnel).

Après l'étape de réaction, le mélange brut obtenu a été neutralisé en discontinu pour éliminer le catalyseur et l'acide acrylique en excès, qui se retrouvent respectivement sous forme de sulfate neutre de butyle et d'acrylate de sodium, dans la phase aqueuse de neutralisation $(A_1)$.

Après addition d'acide sulfurique pour obtenir un rapport molaire $R_H$ = $H^+$/SNBU de 1,5, le nouveau mélange $(A_2)$ est introduit dans le réacteur d'hydrolyse 8 et porté à une température T° de 130°C, pendant un temps $t_R$ de 1,5 heure. Le mélange après réaction est neutralisé jusqu'à pH 8 par NaOH à 8%. La phase $(A_3)$ est ensuite mélangée au mélange synthétique $(S_1)$ pour simuler la composition des eaux usées de l'atelier, et le mélange $(E_1)$ ainsi constitué est introduit en continu sur la colonne de distillation 4 pour récupération du butanol en tête et concentration des eaux épuisées destinées au traitement biologique en pied.

Le rendement d'hydrolyse du sulfate neutre de butyle est de 97%, la DCO dosée sur les eaux épuisées sortant en pied de la colonne 4 est de 6,1 g d'$O_2$ par kg d'eau, soit 15% de la DCO obtenue en l'absence de traitement, dont la quasi-totalité est due à la présence d'acrylate de sodium.

B - En continu

On utilise le même réacteur d'hydrolyse 8 que ci-dessus, mais le mélange réactionnel $(A_2)$ est introduit de façon continue à l'aide d'une pompe, et l'on fait varier la température T° et le temps de séjour $t_R$. Après un temps équivalent à trois fois le renouvellement complet du réacteur, on recueille le mélange hydrolysé $(A_3)$, lequel est ensuite introduit dans l'étape de distillation 4 en lieu et place du mélange $(A_1)$.

Résultats

Pour être encore plus proche des conditions opératoires de l'atelier, on a réalisé le même type de simulation que dans le cas de l'hydrolyse en discontinu, mais avec des étapes neutralisation en continu du mélange brut ($B_1$) après réaction et hydrolyse en continu de la phase aqueuse de neutralisation ($A_1$).

On a fait varier, dans des domaines opératoires proches de ceux qui avaient été déterminés par des essais en tubes (Exemple 4), les paramètres importants de la réaction d'hydrolyse pour en définir les conditions optimales :

- température de la réaction T°
- temps de la réaction $t_R$.

Les résultats de ces essais en continu dans différentes conditions sont résumés dans le Tableau 1 suivant.

Tableau 1

| Hydrolyse du SNBU sur phase aqueuse de neutralisation - Essais en continu | | | | |
|---|---|---|---|---|
| Essai n° | | 1 | 2 | 3 |
| $H^+$/SNBU | mol | 1,5 | 1,5 | 1,5 |
| T° | °C | 130 | 130 | 135 |
| Temps séjour | h | 1,5 | 2 | 2 |
| Rdt hydrolyse | % | 84 | 97 | 96 |
| DCO eaux épuisées | g/kg $O_2$ | | | 8 |

Les rendements d'hydrolyse sont proches pour des temps de séjour de 2 heures (95 à 97%). En revanche, les résultats de l'Essai 1 montrent qu'un temps de séjour inférieur, pour des paramètres égaux par ailleurs (comparaison des Essais 1 et 2), conduit à une efficacité inférieure.

Les eaux d'hydrolyse de l'Essai 3 sont neutralisées (mélange ($A_3$)), puis additionnée au mélange synthétique ($S_1$), et le mélange ($E_1$) ainsi constitué est introduit en continu sur la colonne de distillation 4.

La DCO mesurée sur les eaux épuisées récupérées en pied de colonne est la suivante :

- Essai 3 :

DCO = 8 g d'$O_2$/kg d'eau, par conséquent 19% de la DCO sans hydrolyse.

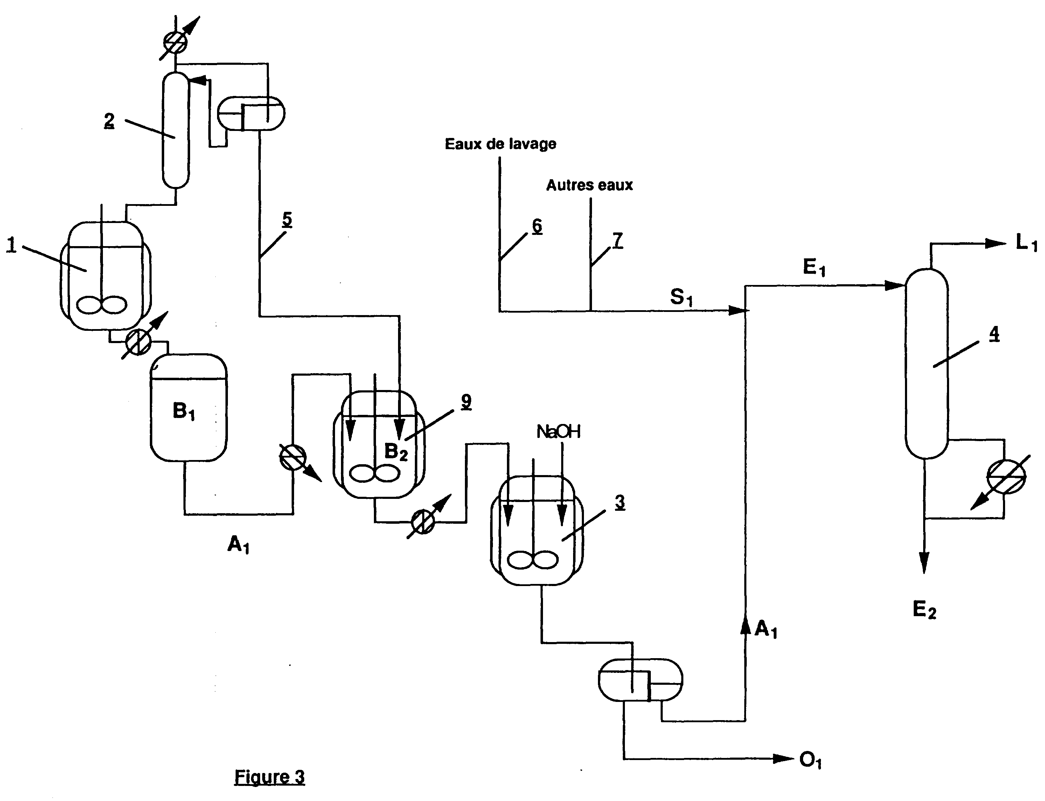

Exemple Comparatif 3 (Fig. 3) - Fabrication d'acrylate de butyle

Pour comparer le procédé selon la présente invention avec le procédé décrit par J. WASILEWSKI et al. dans le passage de littérature précité, consistant en l'hydrolyse du Sulfate de 2-éthyl hexyle dans le milieu réactionnel d'estérification, quelques essais ont été conduits selon cette voie.

Dans un réacteur 9, équipé d'un agitateur mécanique, on introduit le mélange brut ($B_1$) issu de la réaction principale d'estérification, ainsi que la phase aqueuse 5 recueillie dans le mélange azéotropique distillé pendant la réaction (qui représente 12% du mélange ainsi constitué). Ce milieu est porté et maintenu sous agitation à une température T° pendant un temps $t_R$. A l'issue de cette étape, le mélange réactionnel hydrolysé ($B_2$) suit le schéma de purification habituel décrit à l'Exemple de Référence 1 (neutralisation en lieu et place du mélange ($B_1$), récupération de l'alcool).

Résultats

Pour les conditions opératoires suivantes :

- température = 80°C
- durée = 30 minutes
- eau/brut = 11,7%,

les résultats de la simulation complète (hydrolyse du sulfate acide de butyle dans le mélange brut, neutralisation, récupération de l'alcool) indiquent que le traitement entraîne une hydrolyse de 0,5% de l'acrylate de butyle fabriqué par estérification.

Le rendement d'hydrolyse n'est que de 65% avec, pour conséquence, une DCO encore relativement importante dans les eaux épuisées : 21 g d'$O_2$ par kg d'eau, soit seulement 50% par rapport à l'Exemple de Référence 1, sans hydrolyse, dont environ 50% sont dus au sulfate acide de butyle, 35% à l'acide acrylique résiduel en fin de réaction d'estérification et 15% à l'acide acrylique provenant de l'hydrolyse de l'acrylate de butyle.

Exemple 4 : Influence des paramètres de l'hydrolyse acide du sulfate neutre de butyle dans la phase aqueuse ($A_1$)

Cette étude est réalisée, en tubes, sur une solution synthétique neutre de sulfate neutre de butyle. Cette solution est préparée de la manière suivante :

Dans un réacteur agité, chauffé par un chauffe-ballon et équipé d'un réfrigérant et d'un équipement permettant de travailler sous pression réduite, on introduit 740 g de butanol (10 moles) et 202 g d'acide sulfurique à 97% (2 moles). On chauffe sous agitation et reflux pendant 3 heures à une température de 80°C. A l'issue de cette étape réactionnelle, on refroidit, puis l'acidité est neutralisée par une solution de soude 2N, jusqu'à pH = 7-8. On distille le butanol en excès et on dilue à l'eau le sulfate neutre de butyle obtenu jusqu'à une concentration de 5%.

Cette solution aqueuse de sulfate neutre de butyle est utilisée pour étudier l'influence des paramètres opératoires sur la réaction d'hydrolyse :

- quantité d'acide, exprimée en rapport molaire $R_H = H^+/SNBU$ ;
- température de la réaction T° ;
- temps de la réaction $t_R$.

Les essais sont réalisés dans des tubes fermés, plongés dans un bain thermostaté à la température désirée.

Sur le graphe de la Figure 4, on a représenté le rendement d'hydrolyse du sulfate neutre de butyle en fonction de la durée d'hydrolyse, pour des quantités d'acide sulfurique et des températures opératoires différentes. De ce graphe, on a extrait le temps nécessaire pour hydrolyser 90% du sulfate de butyle présent. Les valeurs sont reportées dans le Tableau 2.

Tableau 2

| Hydrolyse du SNBU sur phase aqueuse synthétique - Essai en tubes | | | | | | | | | | |
|---|---|---|---|---|---|---|---|---|---|---|
| $R = H^+/SNBU$ | | 0,5 | 1 | | 2 | | | 4 | | 14 |
| T° | °C | 170 | 150 | 170 | 100 | 120 | 130 | 100 | 130 | 100 |
| t90 | mn | 30 | 55 | 5 | >420 | 205 | 75 | 330 | 185 | 85 |

Exemple 5 de Référence - Fabrication d'acrylate de 2-éthyl hexyle

A - Synthèse du mélange brut réactionnel :

On conduit une estérification dans les mêmes conditions qu'à l'Exemple 1 de Référence avec utilisation du 2-éthyl hexanol. Le taux de transformation de $H_2SO_4$ en SAEH est de 96%. La composition du brut post-réactionnel ($B_1$) est la suivante :

- Acrylate de 2-éthyl hexyle          : 70%
- 2-éthyl hexanol          : 25,7%
- acide acrylique          : 0,5%
- sulfate acide de 2-éthyl hexyle (SAEH)          : 2,15%
- acide sulfurique          : 0,04%
- eau          : 0,2%

B - Purification de l'acrylate de 2-éthyl hexyle

(a) Neutralisation des espèces acides :

De la même manière qu'à l'Exemple 1 de Référence (paragraphe B(a)), les acides présents (SAEH, acide acrylique, acide sulfurique) sont neutralisés par NaOH en léger excès. A l'issue de cette étape, le mélange décante en une phase organique supérieure ($O_1$) et une phase aqueuse ($A_1$).

(b) Récupération de l'alcool

De la même façon que pour la fabrication de l'acrylate de butyle, on alimente la colonne de distillation 4 par un mélange $(E_1)$, constitué par :

- la phase aqueuse $(A_1)$ précitée ; et
- un mélange $(S_1)$ représentatif des autres eaux usées de l'atelier, de composition :

  - eau : 98,9%
  - 2-éthyl hexanol : 0,30%
  - autres composants : 0,8%

Le nouveau mélange $(E_1)$ ainsi constitué est composé de 1 partie de $(A_1)$ pour 3 parties de $(S_1)$.

C - <u>Résultats</u>

A l'issue de l'étape réactionnelle, la totalité de l'acide sulfurique engagé a été transformé en SAEH dans le mélange $(B_1)$, et ce composé passe intégralement dans la phase aqueuse $(A_1)$. Après mélange de cette phase aqueuse avec le mélange synthétique $(S_1)$ pour former le mélange $(E_1)$, les eaux épuisées $(E_2)$ présentent une DCO de 92,2 g d'$O_2$ par kg d'eaux épuisées, dont 72% sont dus à la présence de SAEH, et le complément à la présence d'acrylate de sodium et de traces de 2-éthyl hexyle non distillé.

<u>Exemple 6 de l'Invention (Fig. 2)</u> - Fabrication d'acrylate de 2-éthyl hexyle

On procède comme à l'Exemple 2 : après l'étape de synthèse, le brut obtenu est neutralisé en discontinu pour éliminer le catalyseur et l'acide acrylique en excès, qui se retrouvent respectivement sous forme de SNEH et d'acrylate de sodium dans la phase aqueuse de neutralisation $(A_1)$.

Après addition d'acide sulfurique, pour obtenir un rapport molaire $R_H = H^+/SNEH$ de 1,5, le nouveau mélange $(A_2)$ est introduit dansle réacteur d'hydrolyse et porté à une température T° de 130°C pendant un temps $t_R$ de 1,5 heure. Le mélange après réaction est neutralisé jusqu'à pH 8 par NaOH à 8%. La phase $(A_3)$ est ensuite mélangée à $(S_1)$ (1 partie de $(A_3)$ pour 3 parties de $(S_1)$) pour simuler la composition des eaux usées de l'atelier, et le mélange $(E_1)$ ainsi constitué est introduit en continu sur la colonne de distillation 4 pour récupération du 2-éthyl hexanol en tête et concentration des eaux épuisées destinées au traitement biologique en pied.

Le rendement d'hydrolyse du SNEH est de 87 à 100%, la DCO dosée sur les eaux épuisées sortant en pied de la colonne 4 est de 20,7 g d'$O_2$ par kg d'eaux épuisées, dont la quasi-totalité est due à la présence d'acrylate de sodium et d'alcool résiduel non distillé.

## Revendications

1. Procédé de fabrication d'un (méth)acrylate d'alkyle en $C_1$-$C_{15}$ par estérification directe (en 1) de l'acide (méth) acrylique par l'alcool correspondant, ladite 5 estérification étant catalysée par l'acide sulfurique, le mélange réactionnel brut (B1) obtenu comprenant du (méth) acrylate d'alkyle en $C_1$-$C_{15}$, de l'alcool, de l'acide (méth) acrylique, du sulfate acide d'alkyle en $C_1$-$C_{15}$, des traces d'acide sulfurique et les impuretés habituelles, procédé suivant lequel on fait suivre l'estérification par :

   (a) l'addition (en 3) audit mélange réactionnel brut (B1) d'une base pour neutraliser l'acide (méth)acrylique, le sulfate acide d'alkyle en $C_1$-$C_{15}$, les traces d'acide sulfurique qui y sont présents, les sels résultants passant dans la phase aqueuse (A1) dudit mélange, la phase organique (O1) et la phase aqueuse (A1) issues de cette neutralisation étant séparées et le (méth)acrylate d'alkyle recherché étant récupéré à partir de ladite phase organique (O1) ;
   (b) la récupération de l'alcool (dans A1) à partir de ladite phase aqueuse, les eaux usées (E2) débarrassées dudit alcool étant rejetées,

   caractérisé par le fait qu'on conduit une hydrolyse (en 8) du sulfate acide d'alkyle présent dans la phase aqueuse (A1) issue de la séparation conduite après la neutralisation (en 3), de manière à former dans ladite phase de l'alcool et de l'acide sulfurique, on neutralise les espèces acides résultant de ladite hydrolyse par introduction d'une base dans le milieu, et on adresse la phase aqueuse résultante 3 (A3) à l'étape de récupération de l'alcool.

**2.** Procédé selon la revendication 1, caractérisé par le fait qu'on conduit l'hydrolyse en continu.

**3.** Procédé selon la revendication 1, caractérisé par le fait qu'on conduit l'hydrolyse en discontinu.

**4.** Procédé selon l'une des revendications 1 à 3, caractérisé par le fait qu'on conduit l'hydrolyse en présence d'acide sulfurique comme catalyseur et agent de neutralisation des espèces basiques présentes, ledit acide sulfurique étant introduit dans un rapport molaire $R_H$ du nombre d'équivalents $H^+$ en excès au nombre de moles de sulfate acide d'alkyle au moins égal à 1,5, notamment compris entre 1,5 et 2.

**5.** Procédé selon l'une des revendications 1 à 4, caractérisé par le fait qu'on conduit l'hydrolyse à une température comprise entre 70 et 200°C.

**6.** Procédé selon l'une des revendications 1 à 5, caractérisé par le fait que l'on conduit l'étape de récupération de l'alcool, dans une colonne de distillation (4), l'alcool étant entraîné en tête dans ladite colonne (4).

**7.** Procédé selon l'une des revendications 1 à 6, caractérisé par le fait qu'on conduit l'étape de récupération de l'ester en lavant à l'eau, dans une colonne d'extraction, la phase organique (O1) issue de la séparation de phases qui suit la première neutralisation, puis en adressant le (méth) acrylate d'alkyle lavé à une première colonne à distiller, pour éliminer en tête, les produits légers, recyclés à la réaction d'estérification, et, enfin, en adressant le (méth) acrylate d'alkyle étêté sur une deuxième colonne à distiller, d'où il sort en tête purifié des produits lourds.

**8.** Procédé selon les revendications 6 et 7 prises simultanément, caractérisé par le fait que l'on adresse à la colonne de distillation (4) precitée :

- la phase aqueuse (5) issue de la réaction d'estérification proprement dite ;
- la phase aqueuse (A3) résultant de l'hydrolyse après neutralisation ; et
- la phase aqueuse (6) issue de la colonne d'extraction précitée.

**9.** Procédé selon l'une des revendications 1 à 8, caractérisé par le fait qu'on utilise, comme alcool, le méthanol, le butanol, l'hexanol, le 2-éthyl hexanol.

**Patentansprüche**

**1.** Verfahren zur Herstellung eines $C_1$-$C_{15}$-Alkyl(meth)acrylats durch direkte Veresterung (in 1) von (Meth-)Acrylsäure mit dem entsprechenden Alkohol, wobei die Veresterung durch Schwefelsäure katalysiert wird, die erhaltene Reaktionsrohmischung (B1) das $C_1$-$C_{15}$-Alkyl(meth)acrylat, den Alkohol, (Meth-)Acrylsäure, $C_1$-$C_{15}$-Alkylhydrogensulfat, Spuren von Schwefelsäure und die üblichen Verunreinigungen enthält, und wobei das Verfahren, gemäß dem man die Veresterung durchführt, die folgenden Schritte umfaßt:

(a) Zugabe (in 3) einer Base zu der Reaktionsrohmischung (B1), um die dort vorhandene (Meth-)Acrylsäure, das $C_1$-$C_{15}$-Alkylhydrogensulfat und die Spuren an Schwefelsäure zu neutralisieren, die dort vorhandenen sind, wobei die resultierenden Salze in die wäßrige Phase (Al) dieser Mischung übergehen, die aus dieser Neutralisation stammende organische Phase (01) und wäßrige Phase (A1) getrennt werden und das gewünschte Alkyl(meth)acrylat aus der organischen Phase (01) zurückgewonnen wird;
(b) Wiedergewinnung des Alkohols (in Al) aus der wäßrigen Phase, wobei das gebrauchte, vom Alkohol abgetrennte Wasser (E2) verworfen wird,

dadurch gekennzeichnet, daß man (in 8) das in der wäßrigen Phase (A1) vorhandene Alkylhydrogensulfat hydrolysiert, die aus der nach der Neutralisation (in 3) durchgeführten Trennung stammt, so daß sich in dieser Phase Alkohol und Schwefelsäure bildet, daß man die aus dieser Hydrolyse hervorgehenden sauren Verbindungen durch Zugabe einer Base im Reaktionsmilieu neutralisiert und daß man die resultierende wäßrige Phase (A3) dem Schritt der Alkoholwiedergewinnung zuführt.

**2.** Verfahren nach Anspruch 1, dadurch gekennzeichnet, daß man die Hydrolyse kontinuierlich durchführt.

**3.** Verfahren nach Anspruch 1, dadurch gekennzeichnet, daß man die Hydrolyse diskontinuierlich durchführt.

**4.** Verfahren nach einem der Ansprüche 1 bis 3, dadurch gekennzeichnet, daß man die Hydrolyse in Gegenwart von Schwefelsäure als Katalysator und Neutralisierungsreagenz für die vorhandenen basischen Verbindungen durchführt, wobei die Schwefelsäure in einem Molverhältnis $R_H$ der Anzahl der $H^+$-Äquivalente im Überschuß zur Anzahl der Mole an Alkylhydrogensulfat von mindestens gleich 1,5, insbesondere zwischen 1,5 und 2, hinzugegeben wird.

**5.** Verfahren nach einem der Ansprüche 1 bis 4, dadurch gekennzeichnet, daß man die Hydrolyse bei einer Temperatur zwischen 70 und 200 °C durchführt.

**6.** Verfahren nach einem der Ansprüche 1 bis 5, dadurch gekennzeichnet, daß man den Schritt der Wiedergewinnung des Alkohols in einer Destillationskolonne (4) durchführt, wobei der Alkohol zum Kopf dieser Kolonne (4) geführt wird.

**7.** Verfahren nach einem der Ansprüche 1 bis 6, dadurch gekennzeichnet, daß man den Schritt der Wiedergewinnung des Esters durchführt, indem man in einer Extraktionskolonne die organische Phase (O1) mit Wasser wäscht, die aus der Phasentrennung stammt, die der ersten Neutralisation folgt, dann das gewaschene Alkyl(meth)acrylat einem ersten Destillieraufsatz zuführt, um am Kopf die leichten Produkte zu entnehmen, die der Veresterungsreaktion wieder zugeführt werden, und man schließlich das resultierende Alkyl(meth)acrylat einem zweiten Destillieraufsatz zuführt, von wo es am Kopf von den schweren Produkten gereinigt austritt.

**8.** Verfahren nach den Ansprüchen 6 und 7 gleichzeitig, dadurch gekennzeichnet, daß man der zuvor genannten Destillationskolonne (4) zuführt:

- die wäßrige Phase (5), die aus der eigentlichen Veresterungsreaktion stammt;
- die wäßrige Phase (A3), die aus der Hydrolyse nach der Neutralisation stammt;
- die wäßrige Phase (6), die aus der zuvor genannten Extraktionskolonne stammt.

**9.** Verfahren nach einem der Ansprüche 1 bis 8, dadurch gekennzeichnet, daß man als Alkohol Methanol, Butanol, Hexanol, 2-Ethylhexanol verwendet.

## Claims

**1.** Process for the production of a $C_2$-$C_{15}$ alkyl (meth)acrylate by direct esterification (in 1) of (meth)acrylic acid by the corresponding alcohol, the said esterification being catalysed by sulphuric acid, the crude reaction mixture $(C_1)$ obtained comprising $C_1$-$C_{15}$ alkyl (meth)acrylate, alcohol, (meth)acrylic acid, $C_1$-$C_{15}$ alkyl hydrogen sulphate, traces of sulphuric acid and the usual impurities, according to which process the esterification is followed by:

(a) addition (in 3) to the said crude reaction mixture $(C_1)$ of a base to neutralize the (meth)acrylic acid, the $C_1$-$C_{15}$ alkyl hydrogen sulphate and the traces of sulphuric acid which are present therein, the resulting salts passing into the aqueous phase $(A_1)$ of the said mixture, the organic phase $(O_1)$ and the aqueous phase $(A_1)$ obtained after this neutralization being separated, and the alkyl (meth)acrylate sought being recovered from the said organic phase $(O_1)$;
(b) recovery of the alcohol (in $A_1$) from the said aqueous phase, the used waters $(W_2)$, which are freed of the said alcohol, being expelled,

characterized in that the alkyl hydrogen sulphate which is present in the aqueous phase $(A_1)$ obtained after the separation which is carried out after neutralization (in 3) is hydrolysed (in 8) so as to form the alcohol and sulphuric acid in the said phase, the acidic species resulting from the said hydrolysis are neutralized by introduction of a base into the medium, and the resulting aqueous phase $(A_3)$ is directed to the step for recovery of the alcohol .

**2.** Process according to Claim 1, characterized in that the hydrolysis is carried out in continuous fashion.

**3.** Process according to Claim 1, characterized in that the hydrolysis is carried out in discontinuous fashion.

**4.** Process according to one of Claims 1 to 3, characterized in that the hydrolysis is carried out in the presence of sulphuric acid as catalyst and agent for the neutralization of the basic species present, the said sulphuric acid being introduced in a molar ratio $R_H$, of the number of excess $H^+$ equivalents to the number of moles of alkyl hydrogen sulphate, which is at least equal to 1.5, especially between 1.5 and 2.

**5.** Process according to one of Claims 1 to 4, characterized in that the hydrolysis is carried out at a temperature between 70 and 200°C.

**6.** Process according to one of Claims 1 to 5, characterized in that the step of recovery of the alcohol is carried out in a distillation column (4), the alcohol being entrained at the head in the said column (4).

**7.** Process according to one of Claims 1 to 6, characterized in that the step of recovery of the ester is carried out by washing with water, in an extraction column, the organic phase ($O_1$) obtained after the phase separation which follows the first neutralization, then by directing the washed alkyl (meth)acrylate to a first distillation column, to remove at the head the light products, which are recycled to the esterification reaction, and, finally, by directing the alkyl (meth)acrylate which is freed of this light fraction to a second distillation column, from which it emerges at the head, purified by removal of the heavy products.

**8.** Process according to Claims 6 and 7 taken together, characterized in that the abovementioned distillation column (4) is supplied with:

- the aqueous phase (5) obtained after the actual esterification reaction;
- the aqueous phase ($A_3$) resulting from the hydrolysis after neutralization; and
- the aqueous phase (6) obtained from the abovementioned extraction column.

**9.** Process according to one of Claims 1 to 8, characterized in that the alcohol used is methanol, butanol, hexanol or 2-ethylhexanol.

**Figure 1**

Figure 2

Figure 3

EP 0 609 127 B1

## Cinétique de la réaction d'hydrolyse du SNBU

**Figure 4**